Europäisches Patentamt

**(19)** European Patent Office

Office européen des brevets

**(11)** **EP 0 889 007 A1**

**(12)** **EUROPEAN PATENT APPLICATION**
published in accordance with Art. 158(3) EPC

**(43)** Date of publication:
07.01.1999 Bulletin 1999/01

**(21)** Application number: 97917448.9

**(22)** Date of filing: 21.04.1997

**(51)** Int. Cl.$^6$: **C02F 1/461**

**(86)** International application number:
PCT/JP97/01364

**(87)** International publication number:
WO 98/17588 (30.04.1998 Gazette 1998/17)

**(84)** Designated Contracting States:
**AT BE CH DE DK ES FI FR GB GR IE IT LI LU MC NL PT SE**

**(30)** Priority: 18.10.1996 JP 297407/96

**(71)** Applicant: **MIZ Co., Ltd.**
**Fujisawa-shi Kanagawa 251-087 (JP)**

**(72)** Inventors:
• SATOH, Fumitake
Fujisawa-shi, Kanagawa-ken 252 (JP)
• HAN, Shouka
Fujisawa-shi, Kanagawa-ken 251 (JP)
• YANAGIHARA, Tomoyuki
Yokosuka-shi, Kanagawa-ken 238 (JP)
• NAITOU, Tatsuya
Chigasaki-shi, Kanagawa-ken 253 (JP)

**(74)** Representative:
TER MEER STEINMEISTER & PARTNER GbR
Artur-Ladebeck-Strasse 51
33617 Bielefeld (DE)

**(54)** **REDUCING ELECTROLYTIC WATER AND METHOD FOR PREPARING THE SAME**

**(57)** The present invention provides reducing electrolyzed water which has a pH of 3 to 12 and an oxidation-reduction potential of up to -200 mV, preferably a pH of 5 to 11 and an oxidation-reduction potential of up to -500 mV, and in which the common logarithm of the product of the hydrogen ion concentration $[H^+]$ and the electron concentration $[e^-]$ is at least -4.5, preferably at least 0. The reducing electrolyzed water is used as potable water, agricultural fertilizers, drip solutions and other injections, dialysis solutions and face lotion, and particularly shows significant medical effects.

## FIG. I

EP 0 889 007 A1

**Description**

FIELD OF THE INVENTION

The present invention relates to reducing electrolyzed water obtained by electrolyzing subject water, and potable water, agricultural fertilizers, drip solutions and other injections, dialysis solutions and face lotion which contain the reducing electrolyzed water as their main component, and a method for producing these materials.

BACKGROUND OF THE INVENTION

It has heretofore been reported that use of electrolyzed alkaline water derived by electrolyzing subject water produces the medical effects that extraordinary zymosis and indigestion in the stomach and intestines, diarrhea and gastric hyperacidity are suppressed. The medical effects have been considered to be produced principally by such mineral components contained in electrolyzed alkaline water and present as cations as calcium, sodium, magnesium and potassium.

Electrolyzed alkaline water used for obtaining such medical effects is exclusively defined only by metal ions contained therein and the pH, and is produced by electrolyzing subject water to which calcium, and the like are added until the pH reaches about 9.

However, as a result of carrying out investigations, the present inventors have found that disease is mainly caused by the damage of biomolecules within an organism resulting from oxidation of the biomolecules with active oxygen formed therewithin.

DISCLOSURE OF THE INVENTION

The present inventors have paid attention to that such active oxygen can be reduced with hydrogen to form non-toxic water, and found that electrolytic water having higher medical effects can be obtained if the reaction can be promoted. The present invention has thus been achieved.

Furthermore, since such electrolyzed water has been incapable of being produced by conventional methods, the present inventors have intensively carried out research while paying attention to the reduction reaction rate. As a result, they have discovered that reducing electrolyzed water can be easily formed by selecting and suitably adding some reducing agents and some metal ions, and achieved the present invention.

That is, an object of the present invention is to provide harmless water excellent in reducing capability and a method for producing the same.

In order to achieve the object as mentioned above, the reducing electrolyzed water of the present invention has a pH of 3 to 12 and an oxidation-reduction potential of up to -200 mV, preferably a pH of 5 to 11 and an oxidation-reduction potential of up to -500 mV.

In the present invention, the common logarithm of the product of the hydrogen ion concentration $[H^+]$ and the electron concentration $[e^-]$ is preferably at least -4.5, more preferably at least 0.

As a result of supporting a life by conducting metabolism which utilizes oxygen, an organism forms active oxygen therewithin. The active oxygen has extremely strong oxidizing capability, and oxidizes genes and cells forming the organism. The oxidation is considered to be one cause of disease. The reducing electrolyzed water of the present invention has a significantly high extinction activity for active oxygen, and acts to stabilize active oxygen by accepting active oxygen within the organism.

Furthermore, the reducing electrolyzed water of the present invention can maintain its characteristics for a long period of time, and is excellent in preservability. That is, since the reducing electrolyzed water of the present invention obtained by electrolyzing water containing a reducing agent and/or metal ions contains a reducing agent, the reducing capability of the reducing agent makes the reducing electrolyzed water preserve dissolved oxygen in a trace amount even when the water is exposed to an oxygen environment. Moreover, the reducing electrolyzed water can maintain its oxidation-reduction potential which has become low at a low one for a long period of time, by the action of the metal ions. Accordingly, the reducing electrolyzed water is excellent in preservability.

Furthermore, since the reducing electrolyzed water of the present invention has a pH of 3 to 12, preferably a pH of 5 to 11, namely a large hydrogen ion concentration in spite of its markedly low oxidation-reduction potential, it has strong reducing capability, and has the property that it tends to react with active oxygen within an organism.

Active oxygen $O_2^-$ is reduced by the following reactions to form water:

$$O_2^- + (H^+ + e^-) \rightarrow HO_2^-$$

$$HO_2^- + H^+ \rightarrow H_2O_2 \quad H_2O_2 \rightarrow 2(\cdot OH)$$

$$\cdot OH + (H^+ + e^-) \rightarrow H_2O$$

Accordingly, the chemical equilibria are moved more toward the formation of water $H_2O$ when the hydrogen ion concentration $[H^+]$ and the electron concentration $[e^-]$ are each larger and the product of both concentrations are larger. The hydrogen ion concentration and the electron concentration are expressed as follows:

hydrogen ion concentration $[H^+] = 10^{-pH}$, and

electron concentration $[e^-] = 10^{(-ORP/0.05917)}$.

It can, therefore, be said that when the hydrogen ion concentration $[H^+]$ and the electron concentration $[e^-]$ are each larger and the product of both concentrations are larger, that is, when the common logarithm of the product is larger, the active oxygen is converted into water more. Since the reducing electrolyzed water of the present invention shows the common logarithm of the product of a hydrogen ion concentration $[H^+]$ and an electron concentration $[e^-]$ of at least -4.5, preferably at least 0, it can decrease the active oxygen content.

It is most preferred in the present invention that the dissolved oxygen content be as close to 0 ppm as possible and that the oxidation-reduction potential be as low as possible because inhibition of oxidation by active oxygen within an organism as mentioned above can be expected most.

For reasons as described below, the reducing electrolyzed water of the present invention having such properties is particularly preferably used as the main component of potable water, alcoholic beverages, soft drinks, fruit beverages, fermented lactic drinks, etc., or as drip solutions or other injections, dialysis solutions and toilet water. Use of the reducing electrolyzed water is expected to produce the medical effects that the content of active oxygen which is a byproduct of metabolism utilizing oxygen within an organism can be decreased, and that oxidation of genes and cells can be inhibited.

Furthermore, the reducing electrolyzed water of the present invention is preferably used for agricultural chemicals and agricultural fertilizers other than potable water and injections. Since nitrate nitrogen is used for conventional agricultural fertilizers, field crops contain a large amount of nitrous acid. Since vitamin C decreases in the field crops in inverse proportion to an increase in nitrous acid, the field crops have poor reducing capability. Moreover, when nitrous acid is taken in an organism, it combines with an amine to form a harmful amine nitrite (nitrosoamine). Accordingly, use of the reducing electrolyzed water of the present invention for agricultural chemicals and agricultural fertilizers prevents a decrease in the reducing capability and formation of harmful materials.

The reducing electrolyzed water of the present invention can be produced by electrolyzing water containing a reducing agent. Moreover, it can be produced by electrolyzing water and then adding a reducing agent. Furthermore, it can be produced by electrolyzing water containing a reducing agent, and adding further a reducing agent. Still furthermore, it can be produced by electrolyzing water containing a reducing agent and metal ions. Moreover, it can be produced by electrolyzing water containing a reducing agent and metal ions and adding further metal ions. Furthermore, it can be produced by electrolyzing water and then adding a reducing agent and metal ions. Moreover, it can be produced by electrolyzing water containing a reducing agent and metal ions, and adding further a reducing agent. Furthermore, it can be produced by electrolyzing water containing a reducing agent and adding further a reducing agent and metal ions. Still furthermore, it can be produced by electrolyzing water containing a reducing agent and metal ions and then adding further a reducing agent and metal ions.

It is particularly preferred that the reducing electrolyzed water of the present invention be produced by electrolyzing water containing a reducing agent and metal ions to form electrolyzed alkaline water having a pH of 9 to 12, an oxidation-reduction potential of up to -600 mV and a dissolved oxygen content of up to 3 ppm, and adding a reducing agent to the electrolyzed alkaline water.

Furthermore, it is preferred that the reducing electrolyzed water of the present invention be produced by electrolyzing water containing a reducing agent and metal ions to form electrolyzed alkaline water having a pH of 9 to 12, an oxidation-reduction potential of up to -600 mV and a dissolved oxygen content of up to 3 ppm, and then further electrolyzing the electrolyzed alkaline water.

The reducing agent used in the method for producing the reducing electrolyzed water of the present invention includes a mixture having a g-lactone structure (a cyclic ester obtained by ring closure through cyclodehydration of a carboxylic acid and a hydroxyl group within the molecule) and an OH group, or a saccharide having a five- or six-membered ring containing oxygen and at least one OH group. Examples of the reducing agent include saccharides such as vitamin C, glucose, fructose and lactose, and erythorbic acid (isoascorbic acid).

Furthermore, examples of the reducing agent of the present invention other than the reducing agents mentioned above include oxalacetic acid, vitamin E, EDTA (ethylene-diaminetetraacetic acid) and isopropyl citrate.

Examples of the metal ions used in the method for producing the reducing electrolyzed water of the present invention are all the metal ions. Of the metal ions, particularly preferred examples are sodium ions, potassium ions, calcium ions and magnesium ions.

Preferred examples of the source water to be electrolyzed include ultrapure water, pure water, purified water and distilled water. However, various types of service water may also be used.

When the reducing electrolyzed water of the present invention thus produced is allowed to stand for a long period of time, the oxidation-reduction potential may sometimes increase from -200 to about 0 mV. However, the reducing electrolysed water having an oxidation-reduction potential of up to -200 mV is restored by adding an aqueous solution of a hydroxide such as lithium hydroxide, sodium hydroxide, potassium hydroxide, rubidium hydroxide, cesium hydroxide and calcium hydroxide.

## BRIEF DESCRIPTION OF THE DRAWINGS

Fig. 1 is a cross-sectional view showing an apparatus for producing electrolytic water used in examples of the present invention.

Fig. 2 is a cross-sectional view showing an apparatus for producing electrolytic water used in examples of the present invention.

## PREFERRED EMBODIMENT OF THE PRESENT INVENTION

An example of the present invention will be explained by making reference to drawings.

### Example 1

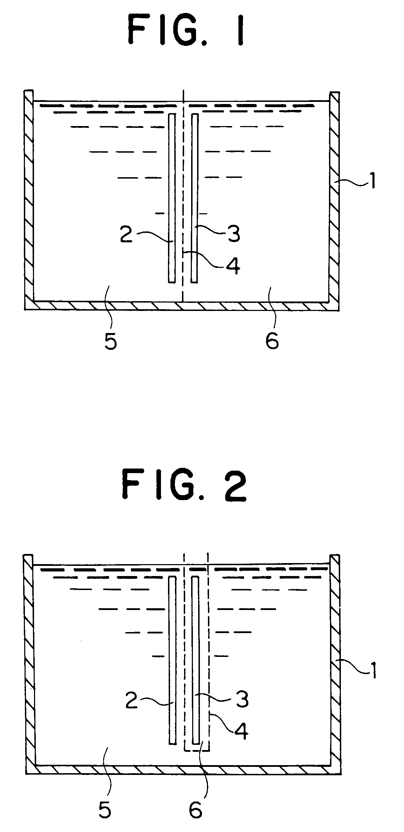

As shown in Fig. 1, in the present example, a diaphragm 4 was arranged in the central portion of an electrolyzer 1 (100 mm wide, 200 mm long and 100 mm high) having a capacity of 2 liters so that the electrolyzer was compartmented to form electrolyzer chambers 5, 6 each having a capacity of 1 liter. Moreover, electrode plates 2, 3 were arranged in the bath in such a manner that one of the principal surface of the electrode plate 2 and one of the principal surface of the electrode plate 3 faced each other, and that a distance L between the electrode plates 2, 3 became 4 mm. Two platinum-plated titanium plates each having a length of 114 mm and a width of 74 mm were used as the electrode plates 2, 3 provided in the electrolyzer 1.

In the present example, water prepared by adding 0.25 g of vitamin C and from 0.01 to 0.02 g of calcium chloride to 2 liters of service water was filled in the electrolyzer 1. The water was electrolyzed by applying a constant voltage of 25 V (maximum current: 2.2 A) between both electrode plates 2, 3 for 20 minutes. Measurements were made on the electrolyzed alkaline water thus produced, and the water was found to have a pH of 10.69, an oxidation-reduction potential (ORP) of -813 mV and a dissolved oxygen content (DO) of 1.47 ppm.

Next, 0.25 g of vitamin C was added to 2 liters of the electrolytic water to give reducing electrolyzed water. The same measurements were made on the water, and the results are shown in Table 1.

In addition, the pH was measured using a pH meter D-13 and a pH sensor #6350-10D (trade names, manufactured by Horiba Ltd.). The ORP was measured using an ORP meter D13 and an ORP sensor #6860-10C (trade names, manufactured by Horiba Ltd.). The DO was measured using a DO meter DO14-P and a DO sensor OE-2102 (trade names, manufactured by Toa Electronics Ltd.).

According to the present example, the ORP and the DO can be made small while a desired pH is being obtained. Moreover, when water is to be produced in accordance with the application, a desired reducing electrolyzed water can be simply obtained, for example, by producing only one type of water having a pH of 9 to 12 and subsequently adding a reducing agent such as vitamin C, without using a specific apparatus after preparation of the one type of water. Furthermore, when a reducing agent is added in a certain ratio before and after electrolysis, the balance of the reducing agent subsequent to the production of the water is improved, and the reducing capability is increased.

### Example 2

Using the same apparatus for producing electrolytic water as in Example 1, water prepared by adding from 0.01 to 0.02 g of calcium chloride to 2 liters of service water was filled in the electrolyzer 1. The water was electrolyzed by applying a constant voltage of 25 V (maximum current: 2.2 A) between both electrode plates 2, 3 for 20 minutes. Measurements were made on the electrolyzed alkaline water thus produced, and the water was found to have a pH of 10.29, an ORP of -790 mV and a DO of 1.00 ppm.

Next, 0.5 g of vitamin C was added to 2 liters of the electrolytic water to give reducing electrolyzed water. The same measurements were made on the water, and the results are shown in Table 1.

According to the present example, the ORP and the DO can be made small while a desired pH is being obtained. Moreover, when a highly reactive reducing agent is used, aging of the reducing capability becomes a problem. In the present example, desired reducing electrolyzed water can be obtained by electrolyzing water containing such metal ions as formed from calcium chloride and adding the reducing agent at the time of using the reducing electrolyzed water. Accordingly, the reducing electrolyzed water is excellent in preserving the reducing capability.

Example 3

Using the same apparatus for producing electrolytic water as in Example 1, water prepared by adding from 0.01 to 0.02 g of calcium chloride to 2 liters of service water was filled in the electrolyzer 1. The water was electrolyzed by applying a constant voltage of 25 V (maximum current: 2.2 A) between both electrode plates 2, 3 for 20 minutes. Measurements were made on the electrolyzed alkaline water thus produced, and the water had a pH of 11.02, an ORP of -840 mV and a DO of 3.00 ppm.

Next, 0.5 g of vitamin C was added to 2 liters of the electrolytic water to give reducing electrolyzed water. The same measurements were made on the water, and the water was found to have a pH of 10.05, an ORP of -770 mV and a DO of 3.00 ppm.

Furthermore, 0.5 g of vitamin C was added to 2 liters of the electrolyzed alkaline water to give reducing electrolyzed water. The same measurements were made on the water, and the results are shown in Table 1.

According to the present example, the ORP and the DO can be made small while a desired pH is being obtained, in the same manner as in Example 2. Moreover, when a highly reactive reducing agent is used, aging of the reducing capability becomes a problem. In the present example, desired reducing electrolyzed water can be obtained by electrolyzing water containing such metal ions as formed from calcium chloride and adding the reducing agent at the time of using the reducing electrolyzed water. Accordingly, the reducing electrolyzed water is excellent in preserving the reducing capability.

Example 4

Using the same apparatus for producing electrolytic water as in Example 1, water prepared by adding 1 g of vitamin C and from 0.01 to 0.02 g of calcium chloride to 2 liters of service water was filled in the electrolyzer 1. The water was electrolyzed by applying a constant voltage of 25 V (maximum current: 2.2 A) between both electrode plates 2, 3 for 20 minutes. Measurements of the pH, the ORP and the DO were made on the reducing electrolyzed water thus produced, and the results are shown in Table 1.

According to the present example, reducing electrolyzed water can be produced most simply.

Example 5

Using the same apparatus for producing electrolytic water as in Example 1, water prepared by adding 1 g of vitamin C and 0.1g of calcium chloride to 2 liters of service water was filled in the electrolyzer 1. The water was electrolyzed by applying a constant voltage of 12 V (maximum current: 0.5 A) between both electrode plates 2, 3 for 3 hours. Measurements of the pH, the ORP and the DO were made on the reducing electrolyzed water thus produced, and the results are shown in Table 1.

According to the present example, reducing electrolyzed water can be produced most simply.

Example 6

First, using the apparatus for producing electrolytic water shown in Fig. 1, water prepared by adding 0.5 g of vitamin C and 0.1 g of sodium chloride to 2 liters of supply water was filled in the electrolyzer 1, and electrolyzed for 40 minutes. Measurements were made on the electrolyzed alkaline water thus produced to find that the water had a pH of 10.03, an ORP of -850 mV and a DO of 0.42 ppm.

Next, using an apparatus for producing electrolytic water shown in Fig. 2, the electrolyzed alkaline water was electrolyzed for 10 minutes by applying a constant voltage of 6 V between two electrodes 2, 3. As shown in Fig. 2, in the apparatus for producing electrolytic water, a pair of electrode plates 2, 3 were arranged in a bath 1 (100 mm wide, 200 mm long and 100 mm high) having a capacity of 2 liters in such a manner that one of the principal surface of the electrode plate 2 and one of the principal surface of the electrode plate 3 faced each other, and a bag-like diaphragm 4 was provided so that the diaphragm surrounded only one of the two electrodes (an anode plate). A distance L between the electrode plates 2, 3 was 4 mm. Two platinum-plated titanium plate each having a length of 50 mm and a width of 6 mm were used as the electrode plates 2, 3 provided in the electrolyzer 1. Measurements of the pH, the ORP and the DO were made on the reducing electrolyzed water thus formed, and the results are shown in Table 1.

According to the present example, the pH can be lowered to about 7 while the ORP is being kept low. Accordingly, the reducing electrolyzed water may be used for injections, etc.

Comparative Example 1

As a comparative example of Example 6, electrolyzed alkaline water produced with the apparatus for producing electrolytic water shown in Fig. 1 and having a pH of 10.03, an ORP of -850 mV and a DO of 0.42 ppm was electrolyzed using the same apparatus. Measurements of the pH, the ORP and the DO were made on the electrolytic water on the acidic side thus produced, and the results are shown in Table 1.

Table 1

|  | PH | ORP(mV) | DO(ppm) |
|---|---|---|---|
| Example 1 | 10.02 | -773 | 1.11 |
| Example 2 | 9.90 | -766 | 1.00 |
| Example 3 | 6.85 | -570 | 1.15 |
| Example 4 | 7.17 | -560 | 1.54 |
| Example 5 | 5.60 | -540 | 0.66 |
| Example 6 | 8.89 | -724 | 0.43 |
| Comparative Example 1 | 6.31 | +45 | 7.56 |

Example 7

Next, using the reducing electrolyzed water of the present invention, the extinction activity for active oxygen within an animal body was evaluated. Specifically, a healthy dog was put on a drip of reducing electrolyzed water having a pH of 6.50, an ORP of -550 mV and a DO of 1.0 ppm for 24 hours. Blood plasma samples were obtained before and after drip. The extinction activity for $O_2^-$ in the samples was measured with an ESR. The blood plasma sample prior to drip showed an extinction activity for $O_2^-$ of 7.8 units/ml, an active oxygen content of 89.20 units/ml and an average value of superoxide dismutase (SOD) of 7.953. On the other hand, the blood plasma sample subsequent to drip showed an increased value of an extinction activity for $O_2^-$ of 15.2 units/ml, a decreased value of an active oxygen content of 58.00 units/ml and an increased value of an average value of SOD of 14.627. That is, it has been confirmed that use of the reducing electrolyzed water of the present invention as a drip solution can decrease the active oxygen content within an animal body.

Example 8

Next, animal test was conducted to confirm the effects of the reducing electrolyzed water on specific disease.
As a first case, a dog infected with lymphoma was put on a drip of 200 ml of the same reducing electrolyzed water as in Example 7. In order to confirm the state of the dog, measurements of the amount of white blood cells (WBCs) and that of platelets (PLTs) which are typical characteristics of the lymphoma were made. The dog infected with lymphoma had an amount of WBCs of 55,800/ml and an amount of PLTs of 39,000/ml before conducting drip. On the other hand, the amount of WBCs decreased to 34,500/ml, and the amount of PLTs increased to 106,000/ml 12 hours after drip. The dog restored its appetite, and showed no difference in appearance compared with other healthy dogs. It has thus been confirmed that use of the reducing electrolyzed water as a drip solution exerts significant effects on the disease of lymphoma.

Example 9

As a second case, a dog infected with brachychronic hepatargia was put on a drip of 720 ml of the same reducing electrolyzed water as in Example 7, and the amounts of albumin (ALB), alkaline phosphatase (ALP), blood urea nitrogen (BUN), cholesterol (CHOL), creatinine (CREA), phosphoric acid (PHOS), total bilirubine (T-Bil), total protein (TP) and globulin (GLOB) which are the typical characteristics of brachychronic hepatargia were measured. The results are shown in Table 2. It has thus been confirmed that use of the reducing electrolyzed water of the present invention as a drip solution exerts significant effects on the disease of brachychronic hepatargia with regard to all the characteristics mentioned above.

Table 2

|  | Prior to drip | 22 hours after drip | 48 hours after drip | 96 hours after drip |
|---|---|---|---|---|
| ALB(g/dl) | 3.89 g/dl | 3.01 | 2.89 | 2.83 |
| ALP(IV) | 562 IV | 348 | 295 | 228 |
| BUN(mg/dl) | 106.5 mg/dl | 26.3 | 4.6 | 1.8 |
| CHOL(mg/dl) | 387.6 mg/dl | 253.6 | 239.1 | 212.4 |
| CREA(mg/dl) | 1.63 mg/dl | 0.54 | 0.6 | 0.46 |
| PHOS(mg/dl) | 11.74 mg/dl | 3.38 | - | 1.99 |
| T-Bil(mg/dl) | 1.22 mg/dl | 0.41 | - | - |
| TP(mg/dl) | 8.76 mg/dl | 6.62 | 6.56 | 6.42 |
| GLOB(mg/dl) | 4.87 mg/dl | 3.62 | 3.67 | 3.59 |

Example 10

As a third case, a dog infected with steroidal hepatitis was put on a drip of 720 ml of the same reducing electrolyzed water as in Example 7 for 72 hours, and the amount of alkaline phosphatase (ALP) which is a typical characteristic of the disease was measured. The amount of ALP was 2,171 units/ml before drip, and the amount decreased to the following values: 1,293 units/ml 8 hours after drip, 912 units/ml 16 hours after drip, 739 units/ml 24 hours after drip, and 621 units/ml 32 hours after drip. It has thus been confirmed that use of the reducing electrolyzed water of the present invention as a drip solution exerts significant effects on the disease of steroidal hepatitis.

It has been confirmed that the reducing electrolyzed water of the present invention has significant medical effects when used as a drink, in addition to use as drip solutions in Examples 7 to 10 mentioned above. As an example, 500 mice were made to drink the reducing electrolyzed water of the present invention for 500 days. The drink exerted no harmful influence on the mice, and the survival rate increased to about 8 times as much with significance. Moreover, the number of T cells increased, and the level of aliphatic hydrogen peroxides in the blood serum lowered. Furthermore, SOD increased.

Example 11

It has been confirmed by the present inventors that the ORP of the reducing electrolyzed water of the present invention increases when allowed to stand still for a long period of time. However, it has also been confirmed that the ORP is recovered to the initial ORP when an aqueous solution containing hydroxide ions are dropwise added. Experiments related to the potential properties of the reducing electrolyzed water of the present invention were conducted.

That is, reducing electrolyzed water having a pH of 5.68 and an ORP of -530 mV was produced by the method of the present invention described above. The water was placed in a container having an open upper end, and allowed to stand still in a room for 20 hours. The pH and the ORP changed to 6.40 and 0 mV, respectively.

A 1 N NaOH solution was added to 200 ml of the reducing electrolyzed water in an amount of 1 to 200 ml, and the changes of the pH and the ORP were measured. The same experiment was repeated twice. The results are shown in Table 3.

Table 3

| 1N-NaOH | Once | | Twice | |
|---|---|---|---|---|
|  | pH | ORP | pH | ORP |
| 1ml | 11.24 | -320mV | 11.07 | -240mV |
| 2ml | 11.73 | -625mV | 11.49 | -659mV |
| 3ml | 12.08 | -659mV | 11.73 | -745mV |
| 4ml | 12.12 | -655mV | 11.89 | -771mV |

Table 3 (continued)

| 1N-NaOH | Once | | Twice | |
|---|---|---|---|---|
| | pH | ORP | pH | ORP |
| 5ml | 12.27 | -628mV | 11.99 | -614mV |
| 6ml | 12.31 | -599mV | 12.07 | -570mV |
| 7ml | 12.37 | -574mV | 12.12 | -535mV |
| 8ml | 12.41 | -534mV | 12.18 | -515mV |
| 9ml | 12.47 | -518mV | 12.24 | -444mV |
| 10ml | 12.50 | -495mV | 12.28 | -384mV |
| 20ml | 12.72 | -485mV | 12.49 | -485mV |
| 30ml | 12.83 | -465mV | 12.64 | -465mV |
| 40ml | 12.92 | -431mV | 12.72 | -431mV |
| 50ml | 13.00 | -415mV | 12.77 | -415mV |
| 60ml | 13.05 | -380mV | 12.82 | -380mV |
| 70ml | 13.08 | -350mV | 12.87 | -350mV |
| 80ml | 13.10 | -342mV | 12.91 | -342mV |
| 90ml | 13.13 | -320mV | 12.94 | -320mV |
| 100ml | 13.06 | -310mV | 13.96 | -310mV |
| 200ml | 13.24 | -290mV | 13.03 | -290mV |

## Example 12

In the same manner as in Example 11, a 1 N KOH solution in an amount of 1 to 200 ml was added to 200 ml of the reducing electrolyzed water which had changed its pH and ORP to 6.40 and 0 mV, respectively when allowed to stand still, and the changes of the pH and ORP were measured. The same experiments were repeated twice. The results are shown in Table 4.

Table 4

| 1N-KOH | Once | | Twice | |
|---|---|---|---|---|
| | pH | ORP | pH | ORP |
| 1ml | 11.00 | -260mV | 10.98 | -225mV |
| 2ml | 11.51 | -666mV | 11.46 | -630mV |
| 3ml | 11.73 | -750mV | 11.71 | -670mV |
| 4ml | 11.88 | -755mV | 11.85 | -650mV |
| 5ml | 11.91 | -665mV | 11.95 | -636mV |
| 6ml | 12.00 | -600mV | 12.02 | -620mV |
| 7ml | 12.09 | -570mV | 12.08 | -565mV |
| 8ml | 12.16 | -530mV | 12.15 | -550mV |
| 9ml | 12.22 | -505mV | 12.21 | -490mV |
| 10ml | 12.28 | -488mV | 12.25 | -480mV |
| 20ml | 12.44 | -485mV | 12.48 | -485mV |

Table 4 (continued)

| 1N-KOH | Once | | Twice | |
|---|---|---|---|---|
| | pH | ORP | pH | ORP |
| 30ml | 12.68 | -460mV | 12.63 | -450mV |
| 40ml | 12.78 | -445mV | 12.72 | -425mV |
| 50ml | 12.87 | -415mV | 12.79 | -420mV |
| 60ml | 12.93 | -380mV | 12.84 | -400mV |
| 70ml | 13.00 | -350mV | 12.90 | -380mV |
| 80ml | 13.03 | -342mV | 12.94 | -370mV |
| 90ml | 13.06 | -320mV | 13.00 | -365mV |
| 100ml | 13.08 | -310mV | 13.07 | -360mV |
| 200ml | 13.17 | -290mV | 13.13 | -330mV |

Example 13

In the same manner as in Example 11, a 1 N $Ca(OH)_2$ solution in an amount of 1 to 7 ml was added to 200 ml of the reducing electrolyzed water which had changed its pH and ORP to 6.40 and 0 mV, respectively when allowed to stand still. The changes of the pH and the ORP were measured. The results are shown in Table 5.

Table 5

| 1N-Ca(OH)$_2$ | pH | ORP |
|---|---|---|
| 1ml | 11.25 | -225mV |
| 2ml | 11.72 | -630mV |
| 3ml | 11.89 | -670mV |
| 4ml | 12.00 | -650mV |
| 5ml | 12.06 | -636mV |
| 6ml | 12.10 | -620mV |
| 7ml | 12.12 | -565mV |

Comparative Example 2

As a comparative Examples 11 to 13, electrolytic water having a pH of 7.43 and an ORP of -180 mV was allowed to stand still in a room for 20 hours. The pH showed no change substantially, and the ORP changed to +250 mV.

A 1 N NaOH solution in an amount of 1 to 200 ml was subsequently added to 200 ml of the electrolytic water, and the changes of the pH and the ORP were measured. The results are shown in Table 6.

Table 6

| 1N-NaOH | pH | ORP(mV) |
|---|---|---|
| 1ml | 11.3 | +70 |
| 5ml | 12.31 | +60 |
| 10ml | 12.65 | +39 |
| 50ml | 13.13 | +12 |
| 100ml | 13.3 | +20 |

It has been clearly confirmed from the results of Tables 3 to 6 that although the characteristic values of electrolytic water such as the pH and the ORP change when it is allowed to stand still for a long period of time, the reducing electrolyzed water of the present invention potentially has the extinction activity for active oxygen, and that even when the apparent characteristic values change, addition of hydroxide ions, etc. can restore the values.

**Claims**

1. Reducing electrolyzed water having a pH of 3 to 12 and an oxidation-reduction potential of up to -200 mV.

2. The Reducing electrolyzed water according to claim 1, wherein the common logarithm of the product of the hydrogen ion concentration $[H^+]$ and the electron concentration $[e^-]$ is at least -4.5.

3. A method for producing reducing electrolyzed water comprising a step of: electrolyzing subject water containing a reducing agent to give reducing electrolyzed water according to claim 1 or 2.

4. A method for producing reducing electrolyzed water comprising the steps of: electrolyzing subject water and adding a reducing agent to give reducing electrolyzed water according to claim 1 or 2.

5. A method for producing reducing electrolyzed water comprising the steps of: electrolyzing subject water containing a reducing agent and adding further a reducing agent to give the reducing electrolyzed water according to claim 1 or 2.

6. A method for producing reducing electrolyzed water comprising a step of: electrolyzing subject water containing a reducing agent and metal ions to give the reducing electrolyzed water according to claim 1 or 2.

7. A method for producing reducing electrolyzed water comprising the steps of: electrolyzing subject water containing a reducing agent and metal ions and adding further metal ions to give the reducing electrolyzed water according to claim 1 or 2.

8. A method for producing reducing electrolyzed water comprising the steps of: electrolyzing subject water and adding a reducing agent and metal ions to give the reducing electrolyzed water according to claim 1 or 2.

9. A method for producing reducing electrolyzed water comprising the steps of: electrolyzing subject water containing a reducing agent and metal ions and adding further a reducing agent to give the reducing electrolyzed water according to claim 1 or 2.

10. A method for producing reducing electrolyzed water comprising the steps of: electrolyzing subject water containing a reducing agent and adding further a reducing agent and metal ions to give the reducing electrolyzed water according to claim 1 or 2.

11. A method for producing reducing electrolyzed water comprising the steps of: electrolyzing subject water containing a reducing agent and metal ions and adding further a reducing agent and metal ions to give the reducing electrolyzed water according to claim 1 or 2.

12. A method for producing reducing electrolyzed water comprising the steps of: electrolyzing subject water containing a reducing agent and metal ions to produce electrolyzed alkaline water having a pH of 9 to 12 and an oxidation-reduction potential of up to -600 mV, and adding a reducing agent to the electrolyzed alkaline water to give the reducing electrolyzed water according to claim 1 or 2.

13. A method for producing reducing electrolyzed water comprising the steps of: electrolyzing subject water containing a reducing agent and metal ions to produce electrolyzed alkaline water having a pH of 9 to 12 and an oxidation-reduction potential of up to -600 mV, and electrolyzing further the electrolyzed alkaline water to give the reducing electrolyzed water according to claim 1 or 2.

14. A method for producing the reducing electrolyzed water according any one of claims 3 to 13, wherein the reducing agent is a mixture having a gamma-lactone structure and containing an OH group or a saccharide having a five- or six-membered ring and at least one OH group.

15. A method for producing the reducing electrolyzed water according any one of claims 3 to 14, wherein the metal ions are sodium ions, potassium ions, calcium ions or magnesium ions.

16. Reducing electrolyzed water obtained by electrolyzing water containing a reducing agent and metal ions, keeping the dissolved oxygen content in a trace amount and maintaining the oxidation-reduction potential at a low level.

17. Reducing electrolyzed water which comes to have an oxidation-reduction potential of up to -200 mV when an aqueous hydroxide solution is added.

18. Potable water comprising the reducing electrolyzed water according to any one of claims 1, 2, 16 and 17 as the principal component thereof.

19. An agricultural fertilizer comprising the reducing electrolyzed water according to any one of claims 1, 2, 16 and 17 as the principal component thereof.

20. An injection comprising the reducing electrolyzed water according to any one of claims 1, 2, 16 and 17.

21. A drip solution comprising the reducing electrolyzed water according to any one of claims 1, 2, 16 and 17.

22. A dialysis solution comprising the reducing electrolyzed water according to any one of claims 1, 2, 16 and 17.

23. Face lotion comprising the reducing electrolyzed water according to any one of claims 1, 2, 16 and 17.

# FIG. 1

# FIG. 2

## INTERNATIONAL SEARCH REPORT

| International application No. |
|---|
| PCT/JP97/01364 |

**A. CLASSIFICATION OF SUBJECT MATTER**

Int. Cl$^6$  C02F1/461

According to International Patent Classification (IPC) or to both national classification and IPC

**B. FIELDS SEARCHED**

Minimum documentation searched (classification system followed by classification symbols)

Int. Cl$^6$  C02F1/46-1/469, C05D9/00-9/02, A61K7/00-7/50, A61K9/08, A61M1/14-1/32

Documentation searched other than minimum documentation to the extent that such documents are included in the fields searched

| | |
|---|---|
| Jitsuyo Shinan Koho | 1926 – 1997 |
| Jitsuyo Shinan Kokai Koho | 1971 – 1997 |
| Toroku Jitsuyo Shinan Koho | 1994 – 1997 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br><br>A | JP, 7-185550, A (Yugen Kaisha Matsuo Seitai Butsuri Kenkyusho),<br>July 25, 1995 (25. 07. 95),<br>Column 1, lines 10 to 14; column 2, line 44 to column 3, line 2 (Family: none) | 1, 2, 18<br>3, 6, 15,<br>16, 19, 23<br>4, 5, 7-14,<br>17, 20-22 |
| X<br>Y<br><br>A | JP, 7-047367, A (Brother Industries, Ltd.),<br>February 21, 1995 (21. 02. 95),<br>Column 3, line 47 to column 4, line 23<br>(Family: none) | 1, 18<br>3, 6, 15,<br>16, 19, 23<br>2, 4, 5,<br>7-14, 17,<br>20-22 |
| Y<br>A | JP, 8-229563, A (Tatsuo Okazaki),<br>September 10, 1996 (10. 09. 96),<br>Column 1, lines 6 to 8 (Family: none) | 3, 6, 15, 16<br>4, 5, 7-14 |
| Y | JP, 6-279157, A (Nippon Karritt K.K.),<br>October 4, 1994 (04. 10. 94),<br>Column 1, lines 2 to 5 (Family: none) | 19 |

[X] Further documents are listed in the continuation of Box C.     [ ] See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| June 30, 1997 (30. 06. 97) | July 8, 1997 (08. 07. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)